# EUROPEAN PATENT APPLICATION

(11) **EP 4 616 804 A1**
(43) Date of publication of application: **17.09.2025**
(21) Application number: 23888457.1
(22) Date of filing: 19.10.2023
(51) Int. Cl.: A61B 5/291, A61B 5/263

(54) **BIOELECTRODE**

(30) Priority: 08.11.2022 JP 2022178656
(71) Applicant: NOK Corporation, Minato-ku Tokyo 105-8585 (JP)
(72) Inventor: KUBO, Masayuki, Fujisawa-shi, Kanagawa 251-0042 (JP); KUROKO, Takanori, Fujisawa-shi, Kanagawa 251-0042 (JP); HAYASHI, Taisei, Fujisawa-shi, Kanagawa 251-0042 (JP); HAYASHI, Takahiro, Fujisawa-shi, Kanagawa 251-0042 (JP)
(74) Representative: Global IP Europe Patentanwaltskanzlei
(86) International application number: PCT/JP2023/037841
(87) International publication number: WO 2024/101106

(57) **Abstract**

A bioelectrode (1) includes a base (21) including an installation surface (21a), and a plurality of electrode protrusions (22) protruding from the installation surface (21a) and including conductive particles and rubber material and being configured to be in contact with a detection target to detect a biometric signal, wherein the plurality of electrode protrusions (22) is disposed in a circular manner on the installation surface, wherein a central axis (O2) of each of the plurality of electrode protrusions (22) is inclined relative to a normal line (O1) passing through a center of the installation surface, wherein a center of a distal end of each of the plurality of electrode protrusions (22) is disposed apart from the center of the installation surface radially more outwardly than a center of a corresponding proximal end, and wherein each of the plurality of electrode protrusions (22) is hollow.

## Description

### TECHNICAL FIELD

This disclosure relates to bioelectrodes.

### BACKGROUND ART

A bioelectrode, which is used to acquire biometric information such as an electroencephalogram, an electrocardiogram, and an electromyogram from a change in electric potential in a living body, is known. As the bioelectrode, a bioelectrode described in Patent Document 1 can be cited, for example.

The bioelectrode described in Patent Document 1 includes a support member and a plurality of electrode members. The support member supports the plurality of electrode members. The plurality of electrode members is a section, which is to be in contact with a body of a subject, and protrudes from the support member in a brush-like manner. In addition, the material of the support member and the plurality of electrode members is conductive rubber. The conductive rubber includes silicone rubber and silver powder.

### Related Art Document

### Patent Document

Patent Document 1: WO 2018/230445

### SUMMARY OF THE INVENTION

### Problem to be Solved by the Invention

A biometric signal regarding an electroencephalogram, etc., is a weak signal of several tens of µV. In general, to detect such a signal with high sensitivity, a large amount of metal powder such as silver powder is used as a material of a bioelectrode. When a large amount of metal powder is used, the material hardness increases and the material cost increases. When the material hardness increases, discomfort may be felt depending on the conditions when the electrode is worn for a long period of time. Thus, there is a possibility that a desired measurement result cannot be obtained.

### Means for Solving Problem

In order to solve the above problems, a bioelectrode according to one aspect of this disclosure includes a base including an installation surface, and a plurality of electrode protrusions protruding from the installation surface and including conductive particles and rubber material, the plurality of electrode protrusions being configured to be in contact with a detection target to detect a biometric signal, wherein the plurality of electrode protrusions is disposed in a circular manner on the installation surface, wherein a central axis of each of the plurality of electrode protrusions is inclined relative to a normal line passing through a center of the installation surface, wherein a center of a distal end of each of the plurality of electrode protrusions is disposed apart from the center of the installation surface radially more outwardly than a center of a corresponding proximal end, and wherein each of the plurality of electrode protrusions is hollow.

In addition, a bioelectrode according to another aspect of this disclosure includes a base including an installation surface, and a plurality of electrode protrusions protruding from the installation surface and including conductive particles and rubber material, the plurality of electrode protrusions being configured to be in contact with a detection target to detect a biometric signal, wherein the plurality of electrode protrusions is disposed in a circular manner on the installation surface, wherein a central axis of each of the plurality of electrode protrusions is inclined relative to a normal line passing through a center of the installation surface, wherein a center of a distal end of each of the plurality of electrode protrusions is disposed apart from the center of the installation surface radially more outwardly than the center of the installation surface, wherein each of the plurality of electrode protrusions includes a semispherical distal portion including the distal end, and a middle portion provided between the distal portion and the installation surface and of a split-cylindrical shape, the middle portion having a split-cylinder shape obtained by cutting off a radially outer portion along the normal line from a cylinder.

### Effect of Invention

According to this disclosure, it is possible to reduce the rigidity of the electrode protrusions and to reduce the possibility that discomfort may be felt when the bioelectrode is worn. In addition, the plurality of electrode protrusions is flexibly bent so as to spread outwardly when the bioelectrode is worn; thus, it is possible to increase the contact area between the electrode protrusions and a measurement portion of a subject. Therefore, it is possible to substantially prevent a decrease in measurement accuracy.

### BRIEF DESCRIPTION OF THE DRAWINGS

Fig. 1 is a side view of a bioelectrode according to a First Embodiment.
Fig. 2 is a bottom view of the bioelectrode according to the First Embodiment.
Fig. 3 is a cross section taken along line A1-A1 of Fig. 2.
Fig. 4 is an enlarged view of a part of a plurality of electrode protrusions shown in Fig. 3.
Fig. 5 is a diagram showing a cross section of a part of an electrode member according to a Second Embodiment.
Fig. 6 is a diagram showing a cross section of a part of an electrode member according to a Third Embodiment.
Fig. 7 is a side view of an electrode member according to a Fourth Embodiment.
Fig. 8 is a bottom view of the electrode member shown in Fig. 7.
Fig. 9 is a cross section of a part of a plurality of electrode protrusions shown in Fig. 7.
Fig. 10 is a side view of a bioelectrode according to a modification.

### MODES FOR CARRYING OUT THE INVENTION

In the following, preferred embodiments according to this disclosure will be described with reference to the accompanying drawings. In addition, the scope of this disclosure is not limited to these embodiments unless the following explanation includes a description that specifically limits this disclosure.

### 1. First Embodiment

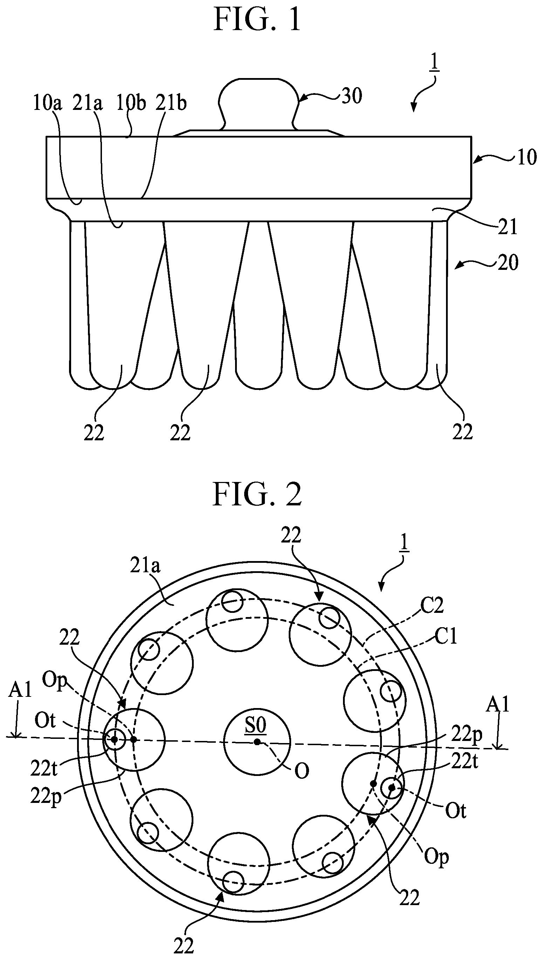

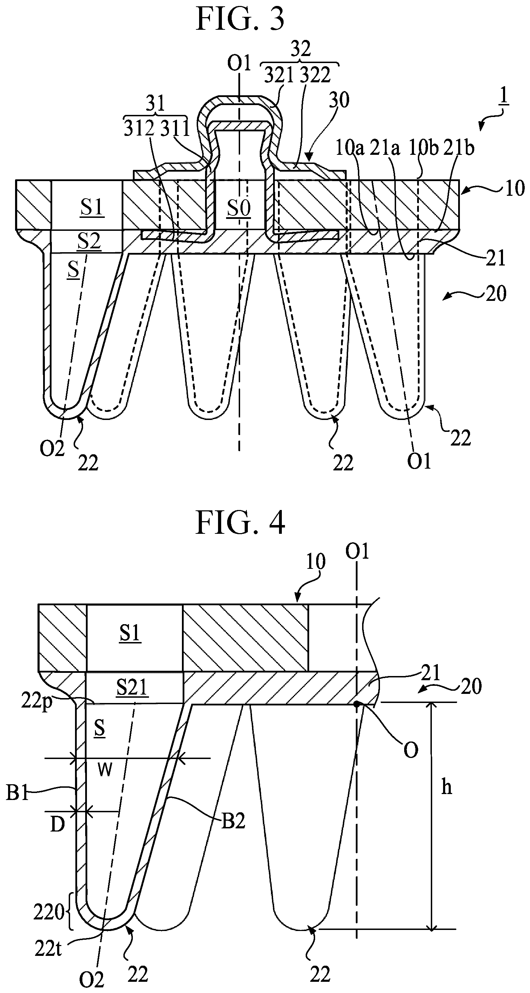

Fig. 1 is a side view of a bioelectrode 1 according to a First Embodiment. Fig. 2 is a bottom view of the bioelectrode 1 according to the First Embodiment. Fig. 3 is a cross section taken along line A1-A1 of Fig. 2.

The bioelectrode 1, which is shown in Fig. 1, Fig. 2, and Fig. 3, is an electrode for detecting, as a biometric signal, a change in electric potential in a living body such as that of a human or an animal. The bioelectrode 1 is used in a state in which it is in contact with a surface of a living body to be measured. Although not shown, the bioelectrode 1 is electrically connected to a measuring device for generating biometric information, such as an electroencephalogram, based on a biometric signal from the bioelectrode 1. In the following, the living body to be measured may be referred to as a subject.

It should be noted that use of the bioelectrode 1 is not limited to taking an electroencephalogram, and it may be measurement of biometric information such as an electrocardiogram or an electromyogram, for example. Thus, a portion of the living body in contact with the bioelectrode 1 is not limited to the scalp, and it may be, for example, an arm, a leg, the chest, or the back or the like, or alternatively, it may be a region not having hair. In addition, use of the bioelectrode 1 is not limited to use for measuring a biometric signal, and it may be a use for providing electrical stimulation to a living body, for example.

As shown in Fig. 1, the bioelectrode 1 includes a support member 10, an electrode member 20, and a connector 30. In a state in which distal ends 22t of a plurality of electrode protrusions 22 included in the electrode member 20 are in contact with a body of a subject, the bioelectrode 1 outputs a biometric signal in the subject from the connector 30. In the following, each of the elements of the bioelectrode 1 will be described sequentially.

The support member 10 is a member for supporting the electrode member 20. The support member 10 is made of an insulating material, in other words, a dielectric material. Examples of the insulating material include a polymer such as silicone rubber, urethane rubber, fluorine rubber, and terpolymer rubber made from ethylene, propylene, and diene. The terpolymer rubber made from ethylene, propylene, and diene is EPDM. From a viewpoint of enhancing adhesion between the support member 10 and the electrode member 20, the insulating material is preferably a polymer of the same type as a polymer constituting the electrode member 20.

In an example shown in Fig. 1, the shape of the support member 10 is a disc shape. The support member 10 includes a support surface 10a and a back surface 10b. The support surface 10a is a surface for supporting the electrode member 20. The back surface 10b is a surface facing in a direction opposite to a direction in which the support surface 10a faces. In addition, as shown in Fig. 3, the support member 10 is provided with a through hole S0 and a plurality of first holes S1. Each of the through hole S0 and the first holes S1 is a hole passing through the support member 10 in a thickness direction, and it is open to each of the support surface 10a and the back surface 10b. As shown in Fig. 2, in plan view, the through hole S0 is provided at the center of the support member 10. In addition, the first holes S1 are provided in one-to-one correspondence with the electrode protrusions 22 included in the electrode member 20 described below. In the drawings, a width of each of the first holes S1 is constant over the entire area in a length direction. In addition, a transverse-sectional shape of each of the first holes S1 is a circle.

It should be noted that in the example shown in Fig. 1, the shape of the support member 10 is a circle in plan view; however, it is not limited thereto, and it may be a polygon, for example. The transverse-sectional shape of each of holes constituted of the through hole S0 and the first holes S1 is not limited to a circle, and it may be a polygon such as a square or pentagon, for example. The support member 10 may be provided, or it may be omitted, as appropriate, or it may be integrated with the electrode member 20.

The electrode member 20 shown in Fig. 1 is a conductive member to be in contact with the surface of the living body. The electrode member 20 is made of an elastic material with conductivity. The elastic material includes rubber material and conductive particles. Examples of the rubber material include silicone rubber, terpolymer rubber made from ethylene, propylene, and diene, nitrile rubber, and urethane rubber and the like. Among them, silicone rubber is suitably used as the rubber material, from a viewpoint of biocompatibility, etc. Examples of the conductive particles include carbon particles, which are made of carbon black, carbon nanotubes, or graphite or the like, metallic particles, which are made of metal such as silver or are made of metal compounds such as silver chloride. It should be noted that the elastic material may include not only the rubber material and the conductive particles, but may also include fiber-based material, such as a nonwoven fabric made of a resin material or a carbon material or the like, or a woven fabric, or it may include various additives.

The electrode member 20 includes a base 21 and the electrode protrusions 22. The base 21 and the electrode protrusions 22 are made of the same material but may be made of different materials separately.

The base 21 is a portion of the electrode member 20 supporting the electrode protrusions 22. In the example shown in Fig. 1, the base 21 is discoid, and in plan view, an outer shape of the base 21 is substantially the same as an outer shape of the support member 10. The base 21 includes an installation surface 21a and a surface 21b facing in a direction opposite to a direction in which the installation surface 21a faces. The surface 21b facing in the direction opposite to the direction in which the installation surface 21a faces is a surface that is in contact with the support surface 10a, and it is bonded by vulcanization bonding or by an adhesive or the like, to the support surface 10a. The installation surface 21a is provided with the electrode protrusions 22.

As shown in Fig. 3, the base 21 is provided with a plurality of second holes S2. The second holes S2 are holes passing through the support member 10 in a thickness direction and are open to each of the installation surface 21a and the surface 21b facing in the direction opposite to the direction in which the installation surface 21a faces. The second holes S2 are provided in one-to-one correspondence with the electrode protrusions 22. In the drawings, a width of each of the second holes S2 is constant over the entire area in a length direction. In addition, a transverse-sectional shape of each of the second holes S2 is a circle.

It should be noted that in the example shown in Fig. 1, the shape of the base 21 is a circle in plan view; however, it is not limited thereto, and it may be a polygon, for example. In addition, the shape of the base 21 in plan view may differ from the shape of the support member 10 in plan view.

Fig. 4 is an enlarged view of a part of the electrode protrusions 22 shown in Fig. 3. As shown in Fig. 4, each of the electrode protrusions 22 is a protrusion protruding from the installation surface 21a of the base 21. Each of the electrode protrusions 22 is made of a single material constituted of a conductive elastic material and functions as an electrode configured to be in contact with a detection target to detect a biometric signal. In addition, each of the electrode protrusions 22 is hollow and includes an internal space S. The internal space S is in communication with a second hole S2 of the base 21 described above. Since each of the electrode protrusions 22 is hollow, compared to a solid protrusion, it is possible to reduce the number of conductive particles. Thus, it is possible to reduce the rigidity of each of the electrode protrusions 22. Consequently, it is possible to reduce the possibility that the subject will experience discomfort when the bioelectrode 1 is worn. In particular, when the bioelectrode 1 is worn for a long period of time, it is less likely to feel discomfort. In addition, since it is possible to reduce the number of conductive particles, it is possible to reduce the material cost.

As shown in Fig. 2, the electrode protrusions 22 are disposed in a circular manner on the installation surface 21a. More specifically, as viewed in a direction of thickness of the base 21, the electrode protrusions 22 are spaced apart from one another at regular angular intervals on a virtual circle C1 with a center O of the installation surface 21a as a center.

Alternatively, the electrode protrusions 22 may be spaced apart from one another at irregular angular intervals. In addition, other multiple electrode protrusions 22 arranged on another circle concentric with the virtual circle C1 may be provided. Furthermore, in an example shown in Fig. 2, the number of electrode protrusions 22 is nine; however it is not limited thereto, and it may be eight or fewer, or it may be ten or more. However, from a viewpoint of realizing stable contact between a living body and the electrode member 20, the number of electrode protrusions 22 is preferably three or more, and more preferably four or more.

As shown in Fig. 4, each of the electrode protrusions 22 includes a proximal end 22p and a distal end 22t. The proximal end 22p of each of the electrode protrusions 22 is coupled to the base 21. The distal end 22t of each of the electrode protrusions 22 is to be in contact with a surface of a living body. In addition, a circle connecting the proximal ends 22p of the respective electrode protrusions 22 to one another is the virtual circle C1 described above. In addition, a circle connecting centers Op of the distal ends 22t of the electrode protrusions 22 to one another is a virtual circle C2. The virtual circle C2 is disposed outside the virtual circle C1. It should be noted that the distal end 22t is not a plane, and the center Ot of the distal end 22t corresponds to the distal end 22t.

As shown in Fig. 2, in each of the electrode protrusions 22, the center Ot of the distal end 22t is disposed apart from the center O of the installation surface 21a radially more outwardly than a center Op of the proximal end 22p. In addition, as shown in Fig. 4, a central axis O2 along a direction of protrusion of each of the electrode protrusions 22 is inclined relative to a normal line O1 passing through the center O of the installation surface 21a. The normal line O1 overlaps a center line of the base 21. This inclination causes the electrode protrusions 22 to be flexibly deformed such that the distal ends 22t first spread radially outwardly when the bioelectrode 1 is pressed against the subject. In addition, as described above, each of the electrode protrusions 22 is hollow; thus, when each of the electrode protrusions 22 is deformed, each of the electrode protrusions 22 is deformed in a bent manner. Consequently, it is possible to increase the contact area of each of the electrode protrusions 22 with a subject. Therefore, it is possible to substantially prevent a decrease in accuracy of measurement by the bioelectrode 1.

An angle of inclination of the central axis O2 relative to the normal line O1 is not particularly limited; in addition, from a viewpoint of ensuring good contact between the living body and the electrode members 20, it is preferably 10 degrees or more, and is more preferably 10 degrees or more and 45 degrees or less, and is still more preferably 10 degrees or more and 30 degrees or less. It should be noted that inclination angles of the electrode protrusions 22 may be the same as, or be different from, one another.

As shown in Fig. 4, each of the electrode protrusions 22 includes a distal portion 220 including the distal end 22t and having a shape of a convex curve. In an example shown in the drawings, the shape of the distal portion 220 is hemispherical. Since the shape of the distal portion 220 of an electrode protrusion 22A is a convex curve, it is possible to reduce the possibility that a subject will experience discomfort when bioelectrode 1 is worn. It should be noted that the shape of the distal portion 220 of each of the electrode protrusions 22 is a convex curve; however, it is not limited thereto, and the shape may be freely selected.

In addition, each of the plurality of electrode protrusion 22 has a tapered shape. Thus, a width W of each of the electrode protrusions 22 tapers from the proximal end 22p toward the distal end 22t. Consequently, the width of the distal portion 220 is less than that of the proximal end 22p. Since the width W of each of the electrode protrusions 22 tapers from the proximal end 22p toward the distal end 22t, compared to a case in which the width W is constant, the electrode protrusions 22 are likely to be deformed so as to spread radially outwardly when the electrode member 20 is pressed against the subject. Thus, it is possible to further increase the contact area of each of the electrode protrusions 22 with a subject. In addition, since the width W tapers from the proximal end 22p toward the distal end 22t, it is possible to maintain the rigidity of the proximal end 22p and a vicinity thereof. Thus, it is possible to substantially prevent deformation of bases of the electrode protrusions 22. Consequently, the bioelectrode 1 can be worn on a measured portion of the subject stably; thus, it is possible to substantially prevent a change in location of the measured portion. It should be noted that widths W of the electrode protrusions 22 may be constant from proximal ends 22p toward distal ends 22t.

As shown in Fig. 4, in this embodiment, a thickness D between an inner wall surface and an outer wall surface of each of the electrode protrusions 22 is constant. Since the thickness D is constant, compared to a case in which the thickness D is not constant, it is possible to easily produce each of the electrode protrusions 22. Furthermore, the thickness D is not particularly limited; in addition, from a viewpoint of ease of deformation, it is preferably 0.2 mm or more and 1.0 mm or less, and more preferably 0.4 mm or more and 0.6 mm or less. It should be noted that thicknesses D of the electrode protrusions 22 may be the same as, or be different from, one another.

In addition, the distal portion 220 of each of the electrode protrusions 22 is provided with no hole that causes the internal space S and the outside to be in communication with each other. If the distal portion 220 is provided with the hole, the rigidity of the distal portion 220 is reduced beyond that which is necessary. As a result, when the distal portion 220 of each of the electrode protrusions 22 is pressed against a subject, the distal portion 220 is likely to collapse. Thus, there is a high possibility that a portion of each of the electrode protrusions 22, which is interposed between the distal portion 220 and the proximal end 22p, is not deformed in a bent manner and only the distal portion 220 is collapsed. In this case, an increase in the contact area of each of the electrode protrusions 22 with the subject is not expected. In contrast, since each of the electrode protrusions 22 according to this embodiment is not provided with the hole, the electrode protrusions 22 are likely to be deformed so as to spread radially outwardly; thus, it is possible to further increase the contact area of each of the electrode protrusions 22 with the subject.

In this embodiment, a transverse-sectional outer shape of each of the electrode protrusions 22 is a circle. Thus, compared to a case in which the transverse-sectional outer shape of each of the electrode protrusions 22 is a quadrilateral, it is possible to reduce the possibility that subject will feel discomfort. It should be noted that the transverse-sectional outer shape of each of the electrode protrusions 22 is not limited to a circle and may be a polygon such as a square or a pentagon, for example.

As shown in Fig. 4, in this embodiment, a bus line B1 disposed on a part of each of the electrode protrusions 22 is parallel to the normal line O1, the part of each of the electrode protrusions 22 being the farthest from the normal line O1. In addition, a bus line B2 disposed on a part of each of the electrode protrusions 22 is inclined relative to the normal line O1 such that a distance between the bus line B2 and the normal line O1 gradually increases from the proximal end 22p toward the distal end 22t, the part of each of the electrode protrusions 22 being the closest to the normal line O1.

A height h of an electrode protrusion 22 is not particularly limited, and it is preferably 6 mm or more and 15 mm or less. The height h within such a range allows the electrode protrusion 22 to be in suitable contact with a surface with hair of a living body. If the height h of the electrode protrusion 22 is too short, it may be difficult to maintain a good state of contact between the electrode protrusion 22 and the living body surface with hair depending on a state of the hair of the subject or the like. On the other hand, if the height h of the electrode protrusion 22 is too long, it is undesirable from a viewpoint of reduction in a size of the bioelectrode 1. In addition, when the electrode member 20 is formed by use of a mold, mold releasability tends to be worse.

It should be noted that the height h of the electrode protrusion 22 is a length of a portion of the electrode protrusion 22 from the proximal end 22p to the distal end 22t in the direction of thickness of the base 21, in other words, in a direction along the normal line O1 of the installation surface 21a. In addition, from a viewpoint of ensuring a good state of contact between the living body and the electrode member 20, heights h of the electrode protrusions 22 are preferably equal.

A hardness of a material of each of the electrode protrusions 22, in other words, a hardness of a conductive material including the conductive particles and the rubber material is not particularly limited; in addition, from a viewpoint of ease of deformation, it is preferably 40 degrees or more and 80 degrees or less measured by use of a durometer "Type A" according to JIS K 6253.

The connector 30 shown in Fig. 3 is a snap-fastener type of male connector and is connected by fitting to a female connector electrically connected to the measuring device, although this is not shown. The connector 30 includes a first conductive member 31 and a second conductive member 32. Each of the first conductive member 31 and the second conductive member 32 is a cylinder with a bottom, which has a flange, and is made of a metallic material such as stainless steel, for example. **In** addition, the first conductive member 31 and the second conductive member 32 are fitted to each other.

The first conductive member 31 includes a first cylinder with a bottom 311 and a first flange 312. The first cylinder with a bottom 311 substantially has a shape of a cylinder with a bottom. The first cylinder with a bottom 311 includes an open end coupled to the first flange 312. A part of the first cylinder with a bottom 311 is inserted in the through hole S0 of the support member 10. The first flange 312 is in contact with the support surface 10a of the support member 10 and has a shape of a flange extending radially outwardly from the open end of the first cylinder with a bottom 311.

The second conductive member 32 includes a second cylinder with a bottom 321 and a second flange 322. The second cylinder with a bottom 321 substantially has a shape of a cylinder with a bottom. The second cylinder with a bottom 321 includes an open end coupled to the second flange 322. An inner diameter of the second cylinder with a bottom 321 is substantially equal to an outer diameter of the first cylinder with a bottom 311. The second cylinder with a bottom 321 is disposed so as to cover a part of the first cylinder with a bottom 311, and the first conductive member 31 and the second conductive member 32 are fitted to each other. The second flange 322 is in contact with the back surface 10b of the support member 10 and has a shape of a flange extending radially outwardly from the open end of the second cylinder with a bottom 321. In a state in which the first conductive member 31 and the second conductive member 32 are fitted to each other, the support member 10 is sandwiched between the first flange 312 and the second flange 322. Thus, the connector 30 is fixed to the support member 10.

As described above, in the bioelectrode 1 according to this embodiment, each of the electrode protrusions 22 is made of a single material including the conductive particles and the rubber material and it is solid; in addition, the central axis O2 of each of the electrode protrusions 22 is inclined relative to the normal line O1 so as to extend radially. Thus, it is possible to reduce the rigidity of each of the electrode protrusions 22; consequently, it is possible to reduce the possibility that discomfort is felt when the bioelectrode 1 is worn. In addition, each of the electrode protrusions 22 is flexibly bent so as to spread outwardly when the bioelectrode 1 is worn; thus, it is possible to increase the contact area between each of the electrode protrusions 22 and a measurement portion of a subject. Therefore, it is possible to substantially prevent a decrease in measurement accuracy.

### 2. Second Embodiment

In the following, a Second Embodiment according to this disclosure will be described. In the Second Embodiment, for elements having effects and functions substantially the same as those of the First Embodiment, reference signs as used in the descriptions of the First Embodiment are used, and detailed explanations of such elements are omitted as appropriate.

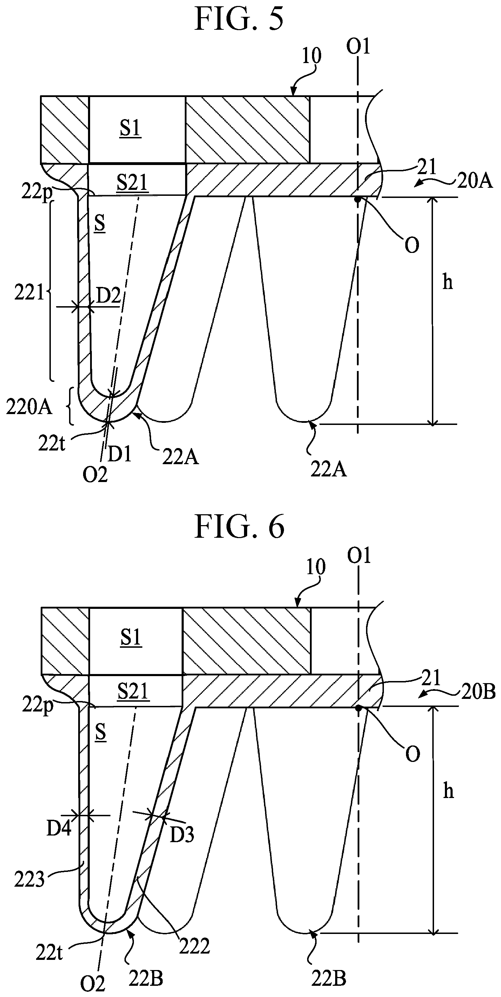

Fig. 5 is a diagram showing a cross section of a part of an electrode member 20A according to the Second Embodiment. As shown in Fig. 5, the Second Embodiment is the same as the First Embodiment, except that a thickness between an inner wall surface and an outer wall surface of an electrode protrusion 22A, that is, any one of electrode protrusions 22A included in the electrode member 20A, is not constant.

As shown in Fig. 5, the electrode protrusion 22A includes a distal portion 220A and a thin portion 221. The distal portion 220A includes a distal end 22t and is a portion of the electrode protrusion 22A having a shape of a convex curve. The thin portion 221 is a portion of the electrode protrusion 22A disposed between the distal portion 220A and the installation surface 21a, the portion of the electrode protrusion 22A having a thickness D2 less than a thickness D1 of the distal portion 220A.

Since the thickness D1 of the distal portion 220A is greater than the thickness D2 of the thin portion 221, it is possible to increase the strength of the distal portion 220A. Thus, the electrode protrusion 22A, which is any one of the electrode protrusions 22A, is likely to be deformed not from the distal portion 220A, but from a vicinity of the middle of the electrode protrusion 22A that is in a direction of protrusion of the electrode protrusion 22A. In other words, it is possible to substantially prevent collapse of the distal portion 220A and to easily deform each of the electrode protrusions 22A in a bent manner. Thus, it is possible to increase the contact area of each of the electrode protrusions 22A with the subject. Consequently, it is possible to improve measurement accuracy.

The thickness D1 is not particularly limited; in addition, from a viewpoint of substantially preventing collapse of the distal portion 220A, it is preferably 1 mm or more and 5 mm or less, and preferably 2 mm or more and 3 mm or less. In addition, the thickness D2 is not particularly limited; in addition, from a viewpoint of ease of deformation, it is preferably 0.2 mm or more and 1.0 mm or less, and more preferably 0.4 mm or more and 0.6 mm or less. It should be noted that thicknesses D1 of the electrode protrusions 22A may be the same as, or be different from, one another, and thicknesses D2 of the electrode protrusions 22A may be the same as, or be different from, one another.

### 3. Third Embodiment

In the following, a Third Embodiment according to this disclosure will be described. In the Third Embodiment, for elements having effects and functions substantially the same as those of the First Embodiment, reference signs as used in the descriptions of the First Embodiment are used, and detailed explanations of such elements are omitted as appropriate.

Fig. 6 is a diagram showing a cross section of a part of an electrode member 20B according to the Third Embodiment. As shown in Fig. 6, the Third Embodiment is the same as the First Embodiment, except that a thickness between an inner wall surface and an outer wall surface of an electrode protrusion 22B, that is, any one of electrode protrusions 22B included in the electrode member 20B, is not constant.

As shown in Fig. 6, the electrode protrusion 22B includes an inner portion 222 and an outer portion 223. The inner portion 222 is a section disposed inside a truncated cone connecting the virtual circle C1 and the virtual circle C2, which are shown in Fig. 2 described above, to each other. On the other hand, the outer portion 223 is a section disposed outside the truncated cone. It should be noted that the inner portion 222 may be referred to as a section disposed radially inwardly apart from a virtual plane connecting central axes O2 of the electrode protrusions 22B shown in Fig. 6. The outer portion 223 may be referred to as a section disposed radially outwardly apart from the virtual plane.

A thickness D4 of the outer portion 223 is less than a thickness D3 of the inner portion 222. Thus, the rigidity of the outer portion 223 can easily be lower than that of the inner portion 222. Consequently, in this embodiment, compared to the First Embodiment, the electrode protrusions 22B are likely to be deformed so as to spread radially outwardly when the electrode member 20B is pressed against the subject. Thus, it is possible to further increase the contact area of each of the electrode protrusions 22B with the subject.

The thickness D3 is not particularly limited; in addition, from a viewpoint of the rigidity of the inner portion 222 being higher than that of the outer portion 223, it is preferably 1 mm or more and 5 mm or less, and more preferably 2 mm or more and 3 mm or less. In addition, the thickness D4 is not particularly limited; in addition, from a viewpoint of ease of deformation, it is preferably 0.2 mm or more and 1.0 mm or less, and more preferably 0.4 mm or more and 0.6 mm or less. It should be noted that thicknesses D3 of the electrode protrusions 22 may be the same as, or be different from, one another, and thicknesses D4 of the electrode protrusions 22 may be the same as, or be different from, one another.

### 4. Fourth Embodiment

In the following, a Fourth Embodiment according to this disclosure will be described. In the Fourth Embodiment, for elements having effects and functions substantially the same as those of the First Embodiment, reference signs as used in the descriptions of the First Embodiment are used, and detailed explanations of such elements are omitted as appropriate.

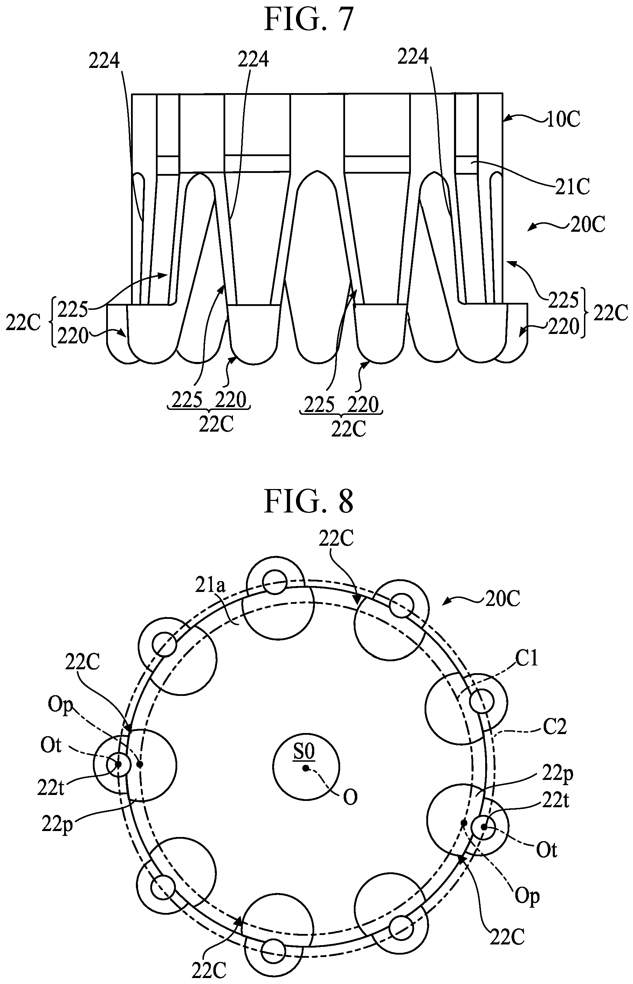

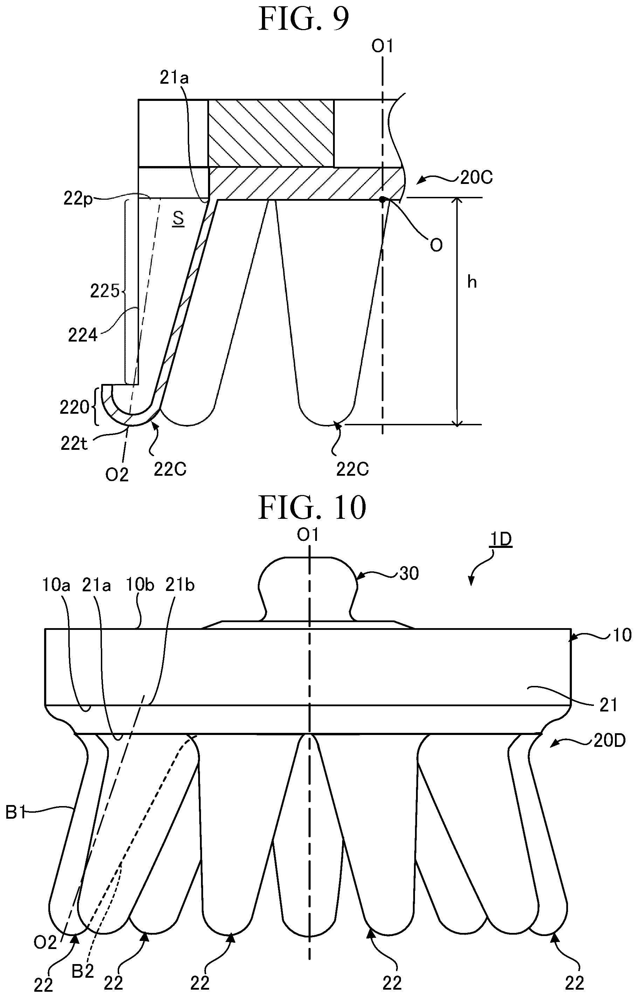

Fig. 7 is a side view of an electrode member 20C according to the Fourth Embodiment. Fig. 8 is a bottom view of the electrode member 20C shown in Fig. 7. Fig. 9 is a cross section of a part of the electrode protrusions 22C shown in Fig. 7. As shown in Fig. 7, Fig. 8, and Fig. 9, the Fourth Embodiment is the same as the First Embodiment, except that a part of an electrode protrusion 22C, that is, any one of electrode protrusions 22C included in the electrode member 20C, is cut off.

As shown in Fig. 7 and Fig. 9, the electrode protrusion 22C that is any one of electrode protrusions 22C includes the distal portion 220 and a middle portion 225. The middle portion 225 is a portion of the electrode protrusion 22C that is any one of electrode protrusions 22C between the distal portion 220 and the installation surface 21a. The shape of the middle portion 225 is a split-cylinder shape obtained by cutting off a radially outer portion along the normal line O1 from the cylinder. In an example shown in the drawings, a transverse-sectional shape of the middle portion 225 along the central axis O2 is an arc. In addition, in this embodiment, the middle portion 225 is disposed radially inwardly apart from the virtual circle C2.

In addition, from another perspective, a part of a side surface of the electrode protrusion 22C that is any one of the electrode protrusions 22C is provided with an opening 224, the part of the side surface of the electrode protrusion 22C being spaced apart from the distal end 22t. The opening 224 is provided radially outwardly so as not to face the normal line O1. The opening 224 is provided from an outer wall surface to an inner wall surface of the electrode protrusion 22 so as to be a through hole that is in communication with a corresponding internal space S.

Since the opening 224 is provided, it is possible to reduce the rigidity of the middle portion 225. In contrast, since the rigidity of the distal portion 220 is maintained, the rigidity of the distal portion 220 can be greater than that of the middle portion 225. Thus, the electrode protrusion 22C, which is any one of the electrode protrusions 22C, is likely to be deformed not from the distal portion 220 but from a vicinity of the middle of the electrode protrusion 22C that is in a direction of protrusion of the electrode protrusion 22C. Consequently, it is possible to increase the contact area of each of the electrode protrusions 22C with the subject. Therefore, it is possible to improve measurement accuracy.

It should be noted that an opening area of the opening 224 is not particularly limited, and it may be freely selected. In addition, opening areas of the electrode protrusions 22C may be the same as, or be different from, one another.

In addition, portions of side surfaces of a base 21c and a support member 10, which correspond to openings 224, are cut off. In addition, in this embodiment, the base 21C and the electrode protrusions 22C are in a single body. Since these are in a single body, compared to a case in which the base 21C and the electrode protrusions 22C are separate from each other, the strength of a mold can be enhanced; thus, it is easy to produce the electrode member 20C with desired rigidity. In addition, in this embodiment, as shown in Fig. 8, a part of the distal portion 220 of each of the electrode protrusions 22C is disposed radially outwardly apart from the base 21; however, as viewed in the direction along the normal line O1, the entire distal portion 220 may overlap the base 21.

### 5. Modifications

The embodiments described above can be modified as described below, for example.

Fig. 10 is a side view of a bioelectrode 1D according to a modification. As shown in Fig. 10, in an electrode member 20D of the bioelectrode 1D, a bus line B1 disposed on a part of each of the electrode protrusions 22D is inclined relative to the normal line O1 such that a distance between the bus line B1 and the normal line O1 gradually increases from the proximal end 22p toward the distal end 22t, the part of each of the electrode protrusions 22D being the farthest from the normal line O1. Similarly, a bus line B2 disposed on a part of each of the electrode protrusions 22D is inclined relative to the normal line O1 such that a distance between the bus line B2 and the normal line O1 gradually increases from the proximal end 22p toward the distal end 22t, the part of each of the electrode protrusions 22D being the closest to the normal line O1.

According to such a bioelectrode 1D, similarly to the respective embodiments, each of the electrode protrusions 22D is flexibly bent so as to spread outwardly when the bioelectrode 1D is worn; thus, it is possible to increase the contact area between each of the electrode protrusions 22D and a measurement portion of a subject.

This disclosure is described based on the preferred embodiments and modifications; however, this disclosure is not limited to the embodiments described above. In addition, a configuration of each element of this disclosure can be replaced with a freely selected configuration that provides functions that are the same as those provided by the embodiments described above, and a freely selected element may be added thereto.

In addition, the respective embodiments may be combined as appropriate. For example, the electrode protrusion 22A according to the Second Embodiment and the electrode protrusion 22B according to the Third Embodiment may be included. In this case, for example, the thickness D1 of the distal portion 220A, the thickness D3 of the inner portion 222, and the thickness D4 of the outer portion 223 may decrease, in this order. In addition, for example, the electrode protrusion 22A according to the Second Embodiment and the electrode protrusion 22C according to the Fourth Embodiment may be included.

### Description of Reference Signs

1... bioelectrode, 1D... bioelectrodes, 10... support member, 10a... support surface, 10b... back surface, 20... electrode member, 20A... electrode member, 20B... electrode member, 20C... electrode member, 20D... electrode member, 21... base, 21C... base, 21a... installation surface, 21b... surface, 22... electrode protrusion, 22A... electrode protrusion, 22B... electrode protrusion, 22C... electrode protrusion, 22D... electrode protrusion, 22p... proximal end, 22t... distal end, 30... connector, 31... first conductive member, 32... second conductive member, 220... distal portion, 220A... distal portion, 221... thin portion, 222... inner portion, 223... outer portion, 224... opening, 225... middle portion, 311...first cylinder with a bottom, 312... first flange, 321...second cylinder with a bottom, 322... second flange, B1... bus line, B2... bus line, C1... virtual circle, C2... virtual circle, D... thickness, D1... thickness, D2... thickness, D3... thickness, D4... thickness, O... center, O1... normal line, O2... central axis, Op... center, Ot... center, S... inner space, S0... through hole, S1... first hole, S2... second hole, h... height.

## Claims

1. A bioelectrode comprising:
a base including an installation surface; and
a plurality of electrode protrusions protruding from the installation surface and including conductive particles and rubber material, the plurality of electrode protrusions being configured to be in contact with a detection target to detect a biometric signal,
wherein the plurality of electrode protrusions is disposed in a circular manner on the installation surface,
wherein a central axis of each of the plurality of electrode protrusions is inclined relative to a normal line passing through a center of the installation surface,
wherein a center of a distal end of each of the plurality of electrode protrusions is disposed apart from the center of the installation surface radially more outwardly than a center of a corresponding proximal end, and
wherein each of the plurality of electrode protrusions is hollow.

2. The bioelectrode according to Claim 1, wherein a width of each of the plurality of electrode protrusions tapers from the proximal end toward the distal end.

3. The bioelectrode according to Claim 1 or 2, wherein each of the plurality of electrode protrusions includes a distal portion including the distal end and having a shape of a convex curve.

4. The bioelectrode according to Claim 1, wherein each of the plurality of electrode protrusions includes:
a distal portion including the distal end; and
a thin portion disposed between the distal portion and the installation surface, the thin portion having a thickness less than a thickness between an inner wall surface and an outer wall surface of the distal portion.

5. The bioelectrode according to Claim 1, wherein each of the plurality of electrode protrusions includes:
an inner portion; and
an outer portion disposed apart from the center of the installation surface radially more outwardly than the inner portion, the outer portion having a thickness less than a thickness between an inner wall surface and an outer wall surface of the inner portion.

6. The bioelectrode according to Claim 1,
wherein a side of each of the plurality of electrode protrusions includes an opening spaced apart from the distal end, and
wherein the opening is provided radially outwardly apart from the center of the installation surface.

7. A bioelectrode comprising:
a base including an installation surface; and
a plurality of electrode protrusions protruding from the installation surface and including conductive particles and rubber material, the plurality of electrode protrusions being configured to be in contact with a detection target to detect a biometric signal,
wherein the plurality of electrode protrusions is disposed in a circular manner on the installation surface,
wherein a central axis of each of the plurality of electrode protrusions is inclined relative to a normal line passing through a center of the installation surface,
wherein a center of a distal end of each of the plurality of electrode protrusions is disposed apart from the center of the installation surface radially more outwardly than the center of the installation surface, and
wherein each of the plurality of electrode protrusions includes:
a semispherical distal portion including the distal end; and
a middle portion provided between the distal portion and the installation surface and of a split-cylindrical shape, the middle portion being open radially outwardly apart from the center of the installation surface.
